# Europäisches Patentamt

## European Patent Office

## Office européen des brevets

(19)

(11) Veröffentlichungsnummer: **0 085 930**
**B1**

(12) # EUROPÄISCHE PATENTSCHRIFT

(45) Veröffentlichungstag der Patentschrift:
30.04.86

(51) Int. Cl.⁴: **A 61 B 17/08**

(21) Anmeldenummer: 83100951.9

(22) Anmeldetag: 02.02.83

(54) Chirurgische Hautklammer.

(30) Priorität: 10.02.82 DE 3204532

(43) Veröffentlichungstag der Anmeldung:
17.08.83 Patentblatt 83/33

(45) Bekanntmachung des Hinweises auf die Patenterteilung:
30.04.86 Patentblatt 86/18

(84) Benannte Vertragsstaaten:
AT BE CH DE FR GB IT LI LU NL SE

(56) Entgegenhaltungen:
GB - A - 2 016 992
GB - A - 2 019 296

(73) Patentinhaber: INTERMEDICAT GMBH,
Gerliswilstrasse 74, CH-6020 Emmenbrücke (CH)

(72) Erfinder: Braun, Karl, Oberer Brühl 5, D-7201 Talheim
(DE)
Erfinder: Fetzer, Jürgen, Mühlstrasse 19,
D-7921 Gerstetten-Dettingen (DE)

(74) Vertreter: von Kreisler, Alek, Dipl.-Chem. et al,
Deichmannhaus am Hauptbahnhof, D-5000 Köln 1 (DE)

## Beschreibung

Die Erfindung betrifft eine chirurgische Hautklammer zum Adaptieren von Wundrändern, mit einem Mittelteil und zu beiden Seiten des Mittelteils angeordneten geraden Schenkeln, von denen Flanken abgehen, welche an ihren freien Enden Spitzen oder Schneiden aufweisen, wobei der Mittelteil als in Richtung der Flankenenden kreisbogenförmig gewölbter Bereich ausgebildet ist, der beim Schließen der Hautklammer zwischen dem Stößel und dem Amboß eines Werkzeugs flachdrückbar ist.

Derartige Hautklammern werden dazu benutzt, die Ränder einer Wunde zusammenzuhalten bis nach ausreichender Ausbildung von Narbengewebe eine sichere Verbindung gegeben ist und die Klammer entfernt werden kann.

Eine chirurgische Hautklammer der eingangs genannten Art (GB-A-2 019 296) weist einen kreisbogenförmig gewölbten mittleren Bereich auf, von dessen Enden in entgegengesetzte Richtungen gerade Schenkel abstehen. Von den Enden der Schenkel ragen rechtwinklig abstehende Flanken, die parallel zueinander verlaufen, in dieselbe Richtung. Beim Einsetzen der Hautklammer in die Haut wird der bogenförmige Bereich gegen einen Amboß des Klammergerätes gedrückt, während Stößel auf die beiden Schenkel derart einwirken, daß diese rechtwinklig zu dem auf dem Amboß flachgedrückten mittleren Bereich verlaufen. Die rechten Winkel zwischen den Flanken und Schenkeln bleiben dabei erhalten. Die Klammer dringt mit diesen rechten Winkeln tief in die Haut ein. Dies ist nicht nur schmerzhaft, sondern führt auch zu Hautspannungen und ungleichmäßigen Wundnähten.

Bei einer weiteren bekannten chirurgischen Hautklammer (GB-A-2 016 992) schließen sich an einen geradlinigen Mittelteil schräg nach hinten weisende Schenkel an, die in nach vorne gebogene seitliche Flanken übergehen. Beim Schließen der Klammer wird der geradlinige Mittelteil gegen einen Amboß gelegt und die zunächst nach hinten weisenden Schenkel werden um nahezu 90° umgebogen, wobei die Flankenenden in die Haut eindringen. Dabei stechen die Klammerspitzen nicht durch das Gewebe, sondern sie reißen durch das zu adaptierende Gewebe der Wundrandzone hindurch. Von jeder der beiden Flanken wird ein großer traumatischer Reißkanal erzeugt. Beim Entklammern kommt noch der Nachteil hinzu, daß die Klammer nicht in ihre Ursprungsform zurückgebogen wird, sondern in der Mitte bogenförmig verformt wird. Die gewinkelten Klammerflanken nehmen nun einen noch ungünstigeren Weg durch das Gewebe und weiten und verletzten das Gewebe erneut. Dieser Vorgang ist mit erheblichen Schmerzen für den Patienten verbunden.

Ein weiterer Nachteil der bekannten Klammern besteht darin, daß sie sich normalerweise nicht für Stapelmagazine mit bogenförmigem Verlauf eignen. Aus gerätetechnischen Gründen ist es bei chirurgischen Klammergeräten oft günstig, ein bogenförmiges Klammermagazin zu verwenden. Für ein solches bogenförmiges Klammermagazin können die bekannten Klammern nicht benutzt werden, da durch ihre im wesentlichen geradlinige Form im Mittelteil eine exakte Führung und ein einfacher Transport mit Federdruck in einem bogenförmigen Magazinkanal nicht möglich ist.

Der Erfindung liegt die Aufgabe zugrunde, eine chirurgische Hautklammer der eingangs genannten Art dahingehend zu verbessern, daß beim Implantieren der Klammer ein atraumatischer Stichkanal entsteht und die Beschädigung des Gewebes auf das unbedingt erforderliche Maß reduziert wird.

Zur Lösung dieser Aufgabe ist erfindungsgemäß vorgesehen, daß die Schenkel, bezogen auf den durch den Mittelpunkt und die Enden des kreisbogenförmigen Mittelteils hindurchgehenden Durchmesser, schräg nach oben und außen gerichtet sind und daß die Flanken mindestens abschnittsweise um den Mittelpunkt des bogenförmigen Mittelteils herum gebogen sind, derart, daß die Flanken beim Schließen der Klammer eine im wesentlichen kreisbogenförmige Einstichbewegung unter Vermeidung von Reißvorgängen ausführen.

Bei der erfindungsgemäßen Hautklammer bewegen sich sowohl beim Klammern als auch beim Entklammern die Klammerspitzen auf einer Kreisbahn. Das Zentrum dieser Bahn fällt in den Bereich des Zentrums des gebogenen Mittelteils. Die gebogenen Klammerflanken folgen der Spitze in der Bewegungsrichtung so, daß der Stichkanal weder verletzt noch aufgeweitet wird. Da das Bahnzentrum der Spitzen und Flanken hinter der Amboßebene liegt, ist ein tiefer Sitz der Klammer im Gewebe gegeben. Der ungünstige Leitereffekt wird hierbei minimiert.

Ein weiterer Vorteil besteht darin, daß die erfindungsgemäße Hautklammer durch den nach innen gebogenen Mittelteil so ausgeführt ist, daß in einem Stapelmagazin die Kraft der Druckfeder auf den Flächenschwerpunkt der Klammer wirken kann. Für den Klammertransport ist daher ein zusätzlicher Schieber nicht erforderlich. Der Klammerstapel läßt sich mühelos auch durch ein gebogenes Klammermagazin schieben.

Da die Flanken mindestens abschnittweise um den Mittelpunkt des bogenförmigen Mittelteils herum gebogen sind, fällt das Zentrum der Bewegungsbahn der Flanken also mit dem Zentrum des gebogenen Mittelteils zusammen. Dadurch wird erreicht, daß mindestens die Endbereiche der Flanken sich auf einer Kreisbahn bewegen und eine reine Stichbewegung durchführen.

Vorzugsweise verläuft jeweils die Tangente an die Mitte der Flanke unter einem Winkel von ca. 60° zu dem zugehörigen Schenkel.

Im Folgenden wird unter Bezugnahme auf die Zeichnungen ein Ausführungsbeispiel der Erfindung näher erläutert.

Es zeigen:
Fig. 1 eine Ansicht der Hautklammer,
Fig. 2 das Ansetzen der Hautklammer über der Inzissionslinie mit dem Werkzeug zum Verformen
Fig. 3 die Hautklammer und das Werkzeug nach dem Schließen,
Fig 4 die Formänderung der Hautklammer während des Schließens,
Fig. 5 eine Ansicht der geklammerten und verschlossenen Wunde, und
Fig. 6 eine Möglichkeit der Klammerführung in einem gebogenen Stapelmagazin.

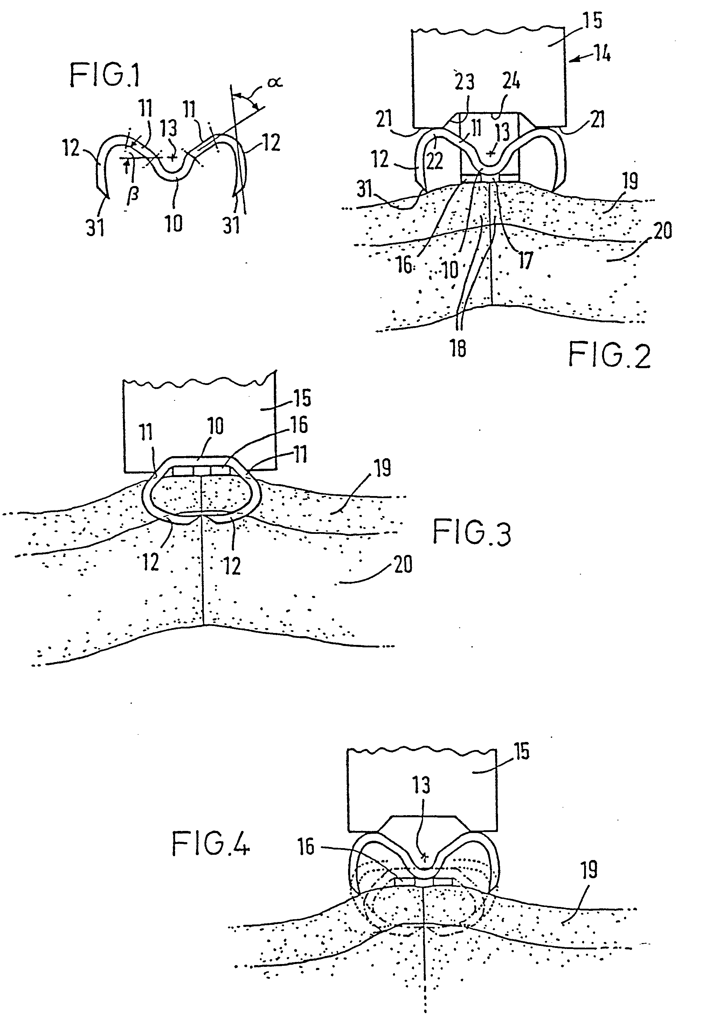

Die in Fig. 1 dargestellte Hautklammer, die aus einem verformbaren Metall besteht, weist einen halbkreisförmige gebogenen Mittelteil 10 auf, von dessen beiden Enden jeweils ein gerader Schenkel 11 unter einem Winkel β von ca. 30° zu dem durch die Enden des Mittelteiles 10 hindurchgehenden Durchmesser schräg nach außen weisend absteht. An jeden Schenkel 11 schließt sich eine flanke 12 an, die bogenförmig ausgebildet ist und mindestens in ihrem äußeren Abschnitt annähernd entlang eines Kreisbogens um den Mittelpunkt 13 des Mittelteiles 10 herum verläuft. Die enden 31 der Flanken 12 sind als Spitzen oder Schneiden ausgebildet. Die Tangente an die Mitte der Flanke 12 verläuft unter einem Winkel α von ca. 60° zu der Längsachse des zugehörigen Schenkels 11. Während die Schenkel 11-bezogen auf den durch den Mittelpunkt 13 und die Enden des Mittelteils 10 hindurchgehenden Durchmesser-Schräg nach oben und außen gerichtet sind, verlaufen die Flanken 12 im wesentlichen senkrecht nach unten, wobei ihre Enden 31 einander zugewandt sind.

In Fig. 2 ist das Ansetzen der Klammer über der Inzissionslinie dargestellt. Die Hautklammer ist in das Werkzeug 14 eingesetzt, welches einen Stößel 15 aufweist, der relativ zu einem Amboß 16 linear bewegbar ist. Der Amboß 16 besteht aus einer flachen Platte, die einen zentrischen Schlitz 17 aufweist, gegen dessen Ränder die Außenseite des Mittelteils 10 der Hautklammer gesetzt wird. Der Amboß 16 liegt an den gegeneinandergesetzten Wundrändern 18 der Hautschicht 19 an. Das Untergewebe der Haut ist mit 20 bezeichnet. Die Enden 13 berühren die obere Hautschicht 19.

Der Stößel 15 weist zwei seitliche Stirnflächen 21 auf, die gegen die bogenförmigen Kniebereiche 22, in denen die Flanken 12 in die Schenkel 11 übergehen, drücken. Die Stirnflächen 21 begrenzen eine trapezförmige Ausnehmung, deren Flanken 23 unter einem Winkel von ca. 45° nach innen und oben gerichtet sind. Die Breite der Basiswand 24 dieser Ausnehmung entspricht der Breite des Ambosses 16 zuzüglich der doppelten Materialstärke der Hautklammer. Die Breite des Ambosses 16 entspricht der Abwicklung des Mittelteiles 10.

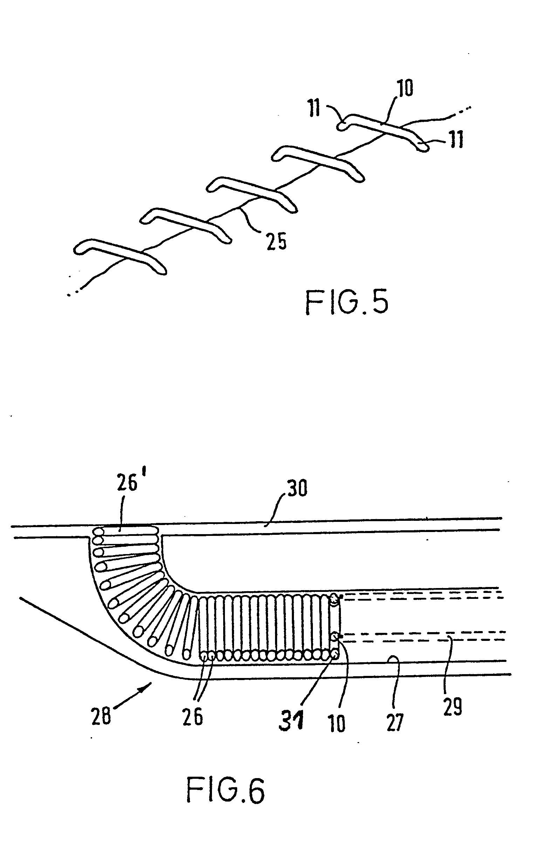

Wenn der Stößel 15 gemäß Fig. 4 nach unten bewegt wird, verformt sich die Hautklammer ausschließlich im Bereich des bogenförmigen Mittelteils 10, welches auf dem Amboß 16 flachgelegt wird, bis die Enden des Mittelteils 10 über die Kanten des Ambosses 16 gebogen werden. Die Flanken 23 des Stößels 15 legen sich schließlich gemäß Fig. 3 gegen die geraden Schenkel 11, die nunmehr schräg nach unten weisen, wobei die Enden der Flanken 12 der Hautklammer gegeneinanderstoßen. Während dieser Verformung der Hautklammer bewegen sich die Flanken 12 gemäß Fig. 4 auf einem Kreisbogen um den Mittelpunkt 13 herum. Auf diese Weise erfolgen von beiden Seiten her kreisbogenförmige Einstichbewegungen in die Hautschicht 19. Es entstehen zwei atraumatische Stichkanäle unter Vermeidung von Reißvorgängen.

Das Werkzeug 14 wird anschließend entfernt, indem der Stößel 15 angehoben und anschließend der Amboß 16 unter dem Mittelteil 10 herausgezogen wird. Fig. 5 zeigt die verschlossene Wunde. Die Wundränder sind gut adaptiert und die Inzissionslinie 25 ist leicht erhaben, um die Schrumpfung bei der Wundheilung zu kompensieren. Die Hautklammern sind durch ihre Form waagerecht orientiert. Dadurch, daß von den jetzt geradegebogenen Mittelteilen 10 die Schenkel 11 schräg nach unten abstehen, ist es möglich, die Hautklammer auf einfache Weise mit einem Entklammergerät zu erfassen und es wird ein Leiterabdruck auf dem Wundbereich verhindert. Die bei der Wundversorgung störenden Klammerstege aus den aus der Haut herausragenden Teilen 10 und 11 sind sehr klein.

Fig. 6 zeigt zahlreiche Hautklammern 26, die in dem Kanal 27 eines Klammermagazins 28 angeordnet sind. Der Kanal 27 enthält eine Druckfeder 29, die im Bereich des gebogenen Mittelteils 10 der letzten Hautklammer angreift.

Der Angriff der Feder 29 erfolgt also im Zentrum der Hautklammern, so daß auch eine gebogene Ausführung des Kanals 27 möglich ist, wobei die Spitzen bzw. Flankenenden 31 nach außen weisen. Die vorderste Hautklammer 26′ liegt in einem querverlaufenden Führungskanal 30, aus dem sie mit einem (nicht dargestellten) Schieber entfernt und dem Werkzeug 14 zugeführt werden kann.

**Patentansprüche**

1. Chirurgische Hautklammer zum Adaptieren von Wundrändern, mit einem Mittelteil (10) und zu beiden Seiten des Mittelteils angeordneten geraden Schenkeln (11), von denen Flanken (12) abgehen, welche an ihren freien Enden Spitzen (31) oder Schneiden aufweisen, wobei der Mittelteil (10) als in Richtung der Flankenenden (31) kreisbogenförmig gewölbter Bereich ausgebildet ist, der beim Schließen der Hautklammer zwischen dem Stößel (15) und dem Amboß (16) eines Werkzeugs (14) flachdrückbar ist, dadurch gekennzeichnet, daß die Schenkel (11), bezogen auf den durch den Mittelpunkt (13) und die Enden des kreisbogenförmigen Mittelteils (10) hindurchgehenden Durchmesser schräg nach oben und außen gerichtet sind und daß die Flanken (12) mindestens abschnittsweise um den Mittelpunkt (13) des bogenförmigen Mittelteils (10) herum gebogen sind, derart, daß die Flanken (12) beim Schließen der Klammer eine im wesentlichen kreisbogenförmige Einstichbewegung unter Vermeidung von Reißvorgängen ausführen.

2. Chirurgische Hautklammer nach Anspruch 1, dadurch gekennzeichnet, daß jeweils die Tangente an die Mitte der Flanke (12) unter einem Winkel ($\alpha$) von ca. 60° zu dem Schenkel (11) verläuft.

3. Chirurgische Hautklammer nach Anspruch 1 oder 2, dadurch gekennzeichnet, daß der Mittelteil (10) annähernd halbkreisförmig ist.

## Claims

1. Surgical clamp for the adaptation of lips of wounds comprising a central portion (10) and straight legs (11) arranged at both sides of said central portion and from which legs extend flanks (12) whose free ends contain points (31) or blades, the central portion (10) being designed to include towards the flank ends a circularly curved region which, during the closing of the clamp, may be flattened between the ram (15) and the anvil (16) of a tool (14),

characterized in that, with reference to the diameter passing through the center (13) and through the ends of the circular central portion (10), the legs (11) are inclined in upward direction and to the outside, and that the flanks (12) are bent at least sectionwise about the center (13) of the arcuate central portion (10), so that, during the closing of the clamp, the flanks (12) perform a substantially circular puncturing movement by avoiding lacerations.

2. Surgical clamp according to claim 1, characterized in that the corresponding tangent to the center of the flank (12) extends at an angle ($\alpha$) of about 60° to the leg (11).

3. Surgical clamp according to claim 1 or 2, characterized in that the central portion (10) is approximately semicircular.

## Revendications

1. Agrafe chirurgicale, pour appliquer l'un contre l'autre les bords d'une blessure, comportant une partie médiane (10) de part et d'autre de laquelle sont disposées des branches rectilignes (11) prolongées respectivement par des flancs (12) comportant à leurs extrémités libres des pointes (31) ou arêtes coupantes; la partie médiane (10) formant un secteur cintré en arc de cercle tourné vers les extrémités (31) des flancs et susceptible d'être aplati par fermeture de l'agrafe entre le poussoir (15) et l'enclume (16) d'un outil (14),

caractérisée en ce que les branches (11) sont dirigées vers le haut et vers l'extérieur, par rapport au diamètre passant par le centre géométrique (13) et par les extrémités de la partie médiane (10) cintrée en arc de cercle, et en ce que les flancs (12) de l'agrafe, au moins sur une partie de leur longueur, sont recourbés en tournant autour du centre géométrique (13) de la partie médiane cintrée (10) de telle sorte que, au moment de la fermeture de l'agrafe, les flancs (12) de l'agrafe suivent un trajet de pénétration sensiblement en arc de cercle susceptible de percer la peau en évitant les effets de déchirure.

2. Agrafe chirurgicale conforme à la revendication 1, caractérisée en ce que la tangente au milieu de chaque flanc (12) de l'agrafe forme avec la branche (11) correspondante un angle ($\alpha$) d'environ 60°.

3. Agrafe chirurgicale conforme à l'une des revendications 1 ou 2, caractérisée en ce que la partie médiane (10) a sensiblement la forme d'un demi-cercle.

FIG.1

FIG.2

FIG.3

FIG.4

FIG.5

FIG.6